# EUROPEAN PATENT SPECIFICATION

(11) **EP 3 432 953 B1**
(45) Date of publication and mention of the grant of the patent: **13.01.2021**
(21) Application number: 17714519.0
(22) Date of filing: 21.03.2017
(51) Int. Cl.: A61M 15/00, A61M 11/04, A24F 47/00, A61M 15/06

(54) **VAPOUR PROVISION DEVICE**
DAMPFBEREITSTELLUNGSVORRICHTUNG
DISPOSITIF DE PRODUCTION DE VAPEUR

(30) Priority: 24.03.2016 GB 201605104
(43) Date of publication of application: 30.01.2019
(73) Proprietor: Nicoventures Trading Limited, London WC2R 3LA (GB)
(72) Inventor: NETTENSTROM, Matthew Joel, London WC2R 3LA (GB); LEADLEY, David, London WC2R 3LA (GB); MCKEON, Thomas Michael, London WC2R 3LA (GB)
(74) Representative: D Young & Co LLP
(86) International application number: PCT/GB2017/050788
(87) International publication number: WO 2017/163051

(56) References cited:
- EP-A1- 1 496 858
- DE-U1-202013 010 929
- US-A1- 2007 052 544
- US-A1- 2010 154 795
- US-A1- 2016 007 654

## Description

### Field

The present disclosure relates to a vapour provision device, e.g. an e-cigarette.

### Background

Many electronic vapour provision systems, such as e-cigarettes and other electronic nicotine delivery systems, are formed from two main components - a cartridge, e.g. a cartomiser, and a control unit. The cartridge generally includes a reservoir of liquid and will often also include an atomiser for vaporising the liquid, although in some cases the atomiser may be included in the control unit. A cartridge that contains an atomiser is sometimes referred to as a cartomiser. The atomiser is often implemented as an electrical (resistive) heater, such as a coil of wire. The control unit generally includes a battery for supplying power to the atomiser. In operation, the control unit may be activated, for example by detecting when a user inhales on the device and/or when the user presses a button, to provide electrical power from the battery to the heater. This activation causes the heater to vaporise a small amount of liquid from the reservoir, which is then inhaled by the user.

This type of e-cigarette therefore generally incorporates two consumables, firstly the liquid to be vaporised, and secondly power in the battery. Regarding the former, once the reservoir of liquid has been exhausted, the cartomiser may be discarded to allow replacement with a new cartomiser. Regarding the latter, the control unit may provide some form of electrical connector for receiving power from an external source, thereby allowing the battery within the e-cigarette to be re-charged.

Although e-cigarettes have developed rapidly over the past few years, there remain areas where it is desirable to improve the operability and user experience for such devices.

US 2007052544 discloses a display system for use in displaying data in respect of usage of a device, the display system including: a detection system capable of detecting events that are indicative of usage of the device; and a display arranged to display data. The display system is arranged to invoke a selected display mode on the display, and being adapted, in response to detection of a given said event, to invoke a selected display mode. The display mode is selectable by the display system from a set of a plurality of different display modes in dependence on an elapsed time between a previous said event and said given event, each of the different display modes identifying a different stage of elapsed time.

US 2010154795 relates to a mouthpiece of a dry powder inhalation device wherein the medicament is packed in the blisters of single dose blister strips. A portion of the air which enters the mouthpiece does not pass through the powder containing blister, but follows an alternative path through the mouthpiece, enabling therefore the modification of the resistance of the device in a simple and cost effective manner.

EP 1946858 relates to a method for the administration of powdered preparations containing tiotropium via inhalation.

US 2016007654 disclose an e-cigarette including: a connecting assembly, a removable mouth piece, an e-liquid tank, a vaporizing assembly, and a rotatable vaporizer tube. The connecting assembly includes: a mouth piece connector, a connecting ring, an e-liquid injection plate, and one or more e-liquid injection holes. The removable mouth piece has a mouth piece plug to be inserted into the mouth piece connector. The e-liquid tank has an e-liquid tank separation plate for dividing e-liquid tank into an upper e-liquid chamber and a lower e-liquid chamber. The e-liquid tank is connected to the connecting assembly. The vaporizing assembly is detachably connected to a lower end of e-liquid tank. The rotatable vaporizer tube is disposed on e-liquid tank separation plate. The rotatable vaporizer tube is insertably connected to the mouth piece through the connecting assembly. The rotatable vaporizer tube is also connected to an air exhaust of the vaporizing assembly.

DE 20201301 0929U discloses a mouthpiece (Drip Tip) with liquid stop for better steam and flavor development, which is designed with a universal interface for different types of evaporators. The Drip Tip a large bore adjacent to the evaporator and at least two smaller holes on the tension side (interface to the mouth). Furthermore, the Drip Tip has a liquid stop which is a barrier that prevents the liquid (liquid / condensate) from entering the mouth via the large bore and the smaller holes. Thus, only steam and no liquid is drawn into the mouth.

### Summary

The invention is defined in the appended claims. The embodiments disclosed herein are considered as examples, unless defined as embodiments of the invention.

Various embodiments provide a vapour provision device including a mouthpiece having a length direction corresponding to the direction of insertion of the vapour provision into the mouth of a user, a width direction corresponding to the direction along the lip-line of the user, and a depth direction corresponding to the opening direction of the lips of the user. The mouthpiece comprises first and second opposing faces and an exit hole located between the first and second opposing faces. The first and second opposing faces are approximately planar and inclined towards one another with respect to the length direction. The first and second opposing faces of the mouthpiece form outer walls of a reservoir for a liquid within the vapour provision device.

In some embodiments, each of the opposing faces has a width which is greater than its length. In some embodiments, each of the opposing faces has a continuously curved section adjacent the exit hole of the mouthpiece, wherein the curvature lies substantially within the plane defined by the longitudinal direction and the width direction. In some embodiments, the mouthpiece further comprises a channel located between the first and second opposing faces, the channel extending in a width direction and having an indentation in the length direction, wherein the exit hole is located in said channel.

### Brief Description of the Drawings

Various embodiments of the disclosure will now be described in detail by way of example only with reference to the following drawings:
Figure 1 is a cross-section through an e-cigarette comprising a cartomiser and a control unit in accordance with some embodiments of the disclosure.
Figure 2 is an isometric external view of the cartomiser of the e-cigarette of Figure 1 in accordance with some embodiments of the disclosure.
Figure 3 is a collection of five external views of the cartomiser of Figure 2 in accordance with some embodiments of the disclosure. In particular, the bottom view shows the cartomiser from underneath, the top view shows the cartomiser from above, the central view shows a face view of the cartomiser (from front or back), and on either side of the central view are respective side views of the cartomiser.
Figure 4 is an exploded view of the cartomiser of the e-cigarette of Figure 1 in accordance with some embodiments of the disclosure.
Figures 5A, 5B and 5C illustrate the wick/heater assembly being fitted into the cartomiser plug in accordance with some embodiments of the disclosure.
Figures 6A and 6B illustrate the inner frame and the vent seal being fitted into the cartomiser plug in accordance with some embodiments of the disclosure.
Figures 7A and 7B illustrate the combination of the inner frame, wick/heater assembly, and primary seal being fitted into the shell and the reservoir then being filled with e-liquid in accordance with some embodiments of the disclosure.
Figures 8A and 8B illustrate the PCB and end cap being fitted to the other components to complete the formation of the cartomiser in accordance with some embodiments of the disclosure
Figure 9 is a top view looking down onto the control unit of the e-cigarette of Figure 1 in accordance with some embodiments of the disclosure
Figures 10A and 10B are cross-sections respectively (a) from side to side, and (b) from front to back, showing the airflow through the e-cigarette of Figure 1 in accordance with some embodiments of the disclosure.

### Detailed Description

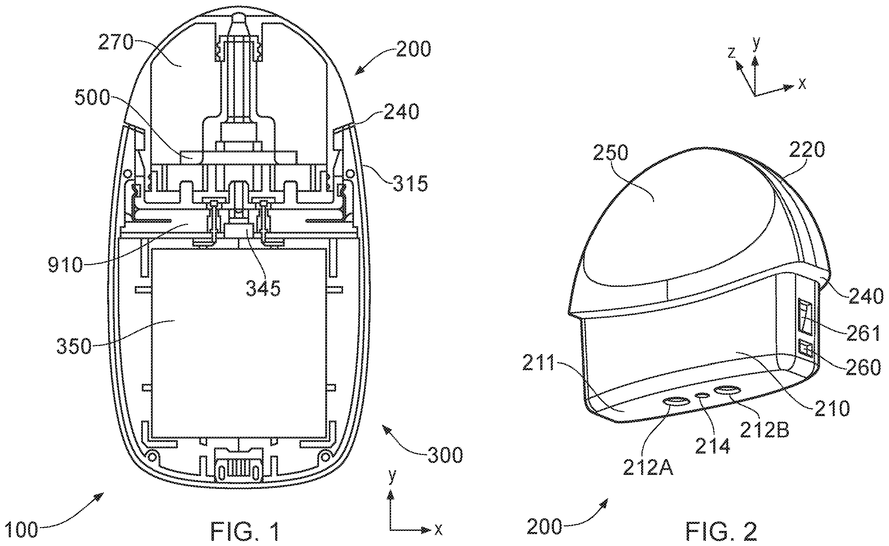

Figure 1 is a cross-section through an e-cigarette 100 in accordance with some embodiments of the disclosure. The e-cigarette comprises two main components, namely a cartomiser 200 and a control unit 300. As discussed in more detail below, cartomiser includes a chamber 270 containing a reservoir of liquid, a heater to act as an atomiser or vaporiser, and a mouthpiece. The liquid in the reservoir (sometimes referred to as the e-liquid) typically includes nicotine in an appropriate solvent, and may include further constituents, for example, to aid aerosol formation, and/or for additional flavouring. The cartomiser 200 further includes a wick/heater assembly 500, which includes a wick or similar facility to transport a small amount of liquid from the reservoir to a heating location on or adjacent the heater. The control unit 300 includes a re-chargeable cell or battery 350 to provide power to the e-cigarette 100, a printed circuit board (PCB) for generally controlling the e-cigarette (not shown in Figure 1), and a microphone 345 for detecting a user inhalation (via a pressure drop). When the heater receives power from the battery, as controlled by the PCB in response to the microphone 345 detecting a user puff on the e-cigarette 100, the heater vaporises the liquid from the wick and this vapour is then inhaled by a user through the mouthpiece.

For ease of reference, the x and y axes are marked in Figure 1. The x axis will be referred to herein as the width of the device (from side to side), while the y axis will be referred to herein as the height axis, where the cartomiser 200 represents the upper portion of the e-cigarette 100 and the control unit 300 represents the lower portion of the e-cigarette. Note that this orientation reflects how a user holds the e-cigarette 100 during normal operation of the device, given that the wick is located in the lower part of the reservoir in the cartomiser 200. Therefore holding the e-cigarette 100 in this orientation ensures that the wick is in contact with liquid at the bottom of the reservoir.

We further assume a z axis (not shown in Figure 1) which is perpendicular to the x and y axes shown in Figure 1. The z axis will be referred to herein as the depth axis. The depth of e-cigarette 100 is significantly less than the width of the e-cigarette, thereby resulting in a generally flat or planar configuration (in the x-y plane). Accordingly, the z axis can be considered as extending from face to face of the e-cigarette 100, where one face may be regarded (arbitrarily) as the front face of the e-cigarette and the opposing face as the back face of the e-cigarette 100.

The cartomiser 200 and the control unit 300 are detachable from one another by separating in a direction parallel to the y-axis, but are joined together when the device 100 is in use so as to provide mechanical and electrical connectivity between the cartomiser 200 and the control unit 300. When the e-liquid in cartomiser reservoir 270 has been depleted, the cartomiser 200 is removed and a new cartomiser is attached to the control unit 300. Accordingly, the cartomiser 200 may sometimes be referred to as the disposable portion of the e-cigarette 100, while the control unit 300 represents the re-usable portion.

Figure 2 is an isometric external view of the cartomiser of the e-cigarette of Figure 1 in accordance with some embodiments of the disclosure. This external view confirms that the depth of the cartomiser 200 (and the e-cigarette 100 as a whole), as measured parallel to the z axis, is significantly less than the width of the cartomiser 200 (and the e-cigarette 100 as a whole), as measured parallel to the x axis. Note that overall, the external appearance of the cartomiser 200 is relatively smooth and uncluttered.

The cartomiser 200 comprises two main portions (at least from an external viewpoint). In particular, there is a lower or base portion 210 and an upper portion 220. The upper portion 220 provides the mouthpiece 250 of the e-cigarette, as described in more detail below. When the cartomiser 200 is assembled with the control unit 300, the base portion 210 of the cartomiser sits within the control unit 300, and hence is not externally visible, whereas the upper portion 220 of the cartomiser protrudes above the control unit 300, and hence is externally visible. Accordingly, the depth and width of the base portion 210 are smaller than the depth and width of the upper portion 220, to allow the base portion to fit within the control unit 300. The increase in depth and width of the upper portion 220 compared with the base portion 210 is provided by a lip or rim 240. When the cartomiser 200 is inserted into the control unit 300, this lip or rim 240 abuts against the top of the control unit.

As shown in Figure 2, the side wall of base portion 210 includes a notch or indentation 260 for receiving a corresponding latching member from the control unit 300. The opposite side wall of the base portion 210 is provided with a similar notch or indentation to likewise receive a corresponding latching member from the control unit 300. It will be appreciated that this pair of notches 260 on the base portion 200 (and the corresponding latching members of the control unit) provide a latch or snap fit connection for securely retaining the cartomiser 200 within the control unit 300 during operation of the device. Adjacent to the notch 260 is a further notch or indentation 261, which is utilised in the formation of the cartomiser 200, as described in more detail below.

As also shown in Figure 2, the bottom wall 211 of the base portion 210 includes two larger holes 212A, 212B on either side of a smaller hole 214 for air inlet. The larger holes 212A and 212B are used to provide positive and negative electrical connections from the control unit 300 to the cartomiser 200. Thus when a user inhales through the mouthpiece 250 and the device 100 is activated, air flows into the cartomiser 200 through the air inlet hole 214. This incoming air flows past the heater (not visible in Figure 2), which receives electrical power from the battery in the control unit 300 so as to vaporise liquid from the reservoir (and more especially from the wick). This vaporised liquid is then incorporated or entrained into the airflow through the cartomiser, and hence is drawn out of the cartomiser 200 through mouthpiece 250 for inhalation by the user.

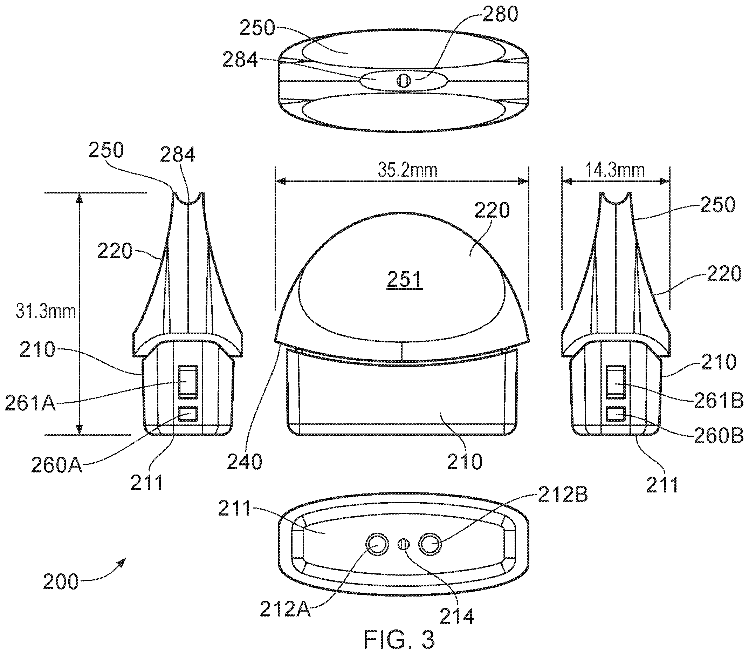

Figure 3 is a collection of five external views of the cartomiser 200 of Figure 2 in accordance with some embodiments of the disclosure. In particular, the bottom view shows the cartomiser from underneath, the top view shows the cartomiser from above, the central view shows a face view of the cartomiser (from front or back), and on either side of the central view are respective side views of the cartomiser. Note that since the cartomiser is symmetric front/back (i.e. with respect to the z axis), the front of the cartomiser and the back of the cartomiser both correspond to the central view of Figure 3. In addition, the cartomiser is also symmetric in the width direction (i.e. with respect to the x axis), hence the two side views to the left and right of the central view are the same.

Figure 3 illustrates the various features of the cartomiser already discussed above with respect to Figure 2. For example, the central view clearly shows the top portion 220 and the bottom portion 210 of the cartomiser. The lower view shows the bottom wall of the base portion 211, including the two larger holes 212A and 212B, which are used to provide positive and negative electrical connections from the control unit 300 to the cartomiser 200, plus the smaller hole 214 for air inlet into the cartomiser. In addition, the two side views show the two notches in each side wall, an upper notch 261A, 261B, and a lower notch 260A, 260B, the latter being used to fasten the cartomiser 200 to the control unit 300.

The top view further shows a hole 280 in the mouthpiece 250 which represents the air outlet from the cartomiser 200. Thus in operation, when a user inhales, air enters the cartomiser at the bottom through inlet 214, flows through the atomiser, including past the heater, where it acquires vapour, and then travels up the centre of the cartomiser to exit through air outlet 280.

Figure 3 provides dimensions of the cartomiser 200, showing a maximum height (in the y direction) of 31.3mm, a maximum width (in the x direction) of 35.2mm, and a maximum depth of 14.3 mm (parallel to the z direction). Note that these maximum width and depth measurements relate to the upper portion 220 of the cartomiser; the width and depth of the base portion 210 are somewhat smaller, in order to allow the base portion to be received into the control unit 300. The difference in width and depth between the upper portion 220 and the base portion 210 is accommodated by the rim or flange 240, as described above.

It will be appreciated that the dimensions shown in Figure 3 are provided by way of example only, and may vary between embodiments. Nevertheless, the dimensions given do confirm that the e-cigarette 100, including the cartomiser, has an approximately flat or planar shape, with one relatively small dimension (the z direction) perpendicular to the planar shape. This planar shape is extended by the control unit 300, which in effect extends the height (y dimension of the cartomiser), but shares substantially the same width and depth.

Figure 3 also gives a clear indication of the size and shape of the mouthpiece 250. In contrast to many e-cigarettes, which provide a circular mouthpiece akin to a straw or conventional cigarette, the mouthpiece 250 has a very different and distinctive shape. In particular, the mouthpiece comprises a pair of large, relatively flat, opposing faces. One of these mouthpiece faces is denoted as face 251 in the central view of Figure 3, and there is a corresponding, opposing face to the rear of the device. (Note that the labelling of front and back for the cartomiser is arbitrary, since it is symmetric with respect to the z axis, and can be fitted either way around onto the control unit 300).

The front and rear faces provide relatively large surfaces onto which the lips of a user can be placed. For example, we can consider the front face to provide a surface for engaging the upper lip, and the rear face to provide a surface for engaging the lower lip. In this configuration, we can regard the height (y axis) of the e-cigarette 100 as defining a longitudinal or length axis extending away from the user's mouth, the width of the e-cigarette 100 (the x axis) as running parallel to the line between a user's upper and lower lips, and the depth of the e-cigarette 100 (the z axis) as running parallel to the direction of separation of the user's upper and lower lips.

The height (or length) of the front and rear mouthpiece faces (approximately 17 mm in the particular embodiment of Figure 3) is broadly comparable to the typical thickness of a lip, and therefore able to readily accommodate a lip placed on the surface. Similarly, the width of the front and rear mouthpiece faces (approximately 28 mm in the particular embodiment of Figure 3) represents a significant proportion (approximately half) of the typical width of lips (from one side of the mouth to the other).

It will be appreciated that these sizings are again provided by way of example only, and may vary according to the particular implementation. Typically the height of the mouthpiece faces 251 is in the range 8-24mm, preferably 12-20mm, while the width of the mouthpiece faces is at least 20mm, and preferably at least 25mm. Typically, the width of the mouthpiece faces is at least 30%, and preferably at least 50%, greater than the length of the mouthpiece faces.

This shape and sizing of the mouthpiece 250 allows the lips of user to engage the mouthpiece for inhalation with much less distortion from the normal resting position of the mouth - e.g. there is no need to purse the lips, as for a straw or conventional cigarette having a small circular mouthpiece. This makes using the mouthpiece 250 of the e-cigarette 100 a more relaxing experience, and also may help to ensure a more consistent seal between the mouth and the mouthpiece.

In addition, e-cigarette 100 (like many other e-cigarettes) uses a sensor to detect airflow through the device, i.e. a user puff, which can then trigger operation of the heater to vaporise the liquid. The device has to discriminate between the airflow caused by a user puff, and other forms of airflow or pressure changes that arise due to other actions or circumstances - e.g. movement of the e-cigarette through the air, being on a railway train which enters a tunnel etc. Having a consistent seal between the mouth and the mouthpiece 250 can help the device provide better discrimination of an actual inhalation, and so reduce the risk of unintentional activation of the heater.

Furthermore, some e-cigarettes use sensor measurements of the airflow through the device not only to initiate activation of the heater, but also to provide dynamic control of the heater (or other components of the e-cigarette). For example, as the measured airflow increases, the heater may be provided with more power, firstly to compensate for the cooling effect of the increased airflow, and/or secondly to vaporise more liquid into the increased airflow. Having a consistent seal between the mouth and the mouthpiece 250 can again help to improve the reliability and accuracy of this dynamic control.

As can be seen in the two side views of Figure 3, the mouthpiece faces 251 are slightly concave along the length direction, i.e. they dip inwards towards the centre of device when progressing along the y axis. These faces 251 may also be slightly concave along the width direction as well. This curvature of the faces helps the lips of a user to rest comfortably on the mouthpiece 250, and hence again assists in providing a good seal between the user's lips and the mouthpiece 250.

In addition, with reference to the side views of Figure 3, it can be seen that the front and back faces of the mouthpiece generally slope towards one another at the top of the device. In other words, the depth or separation of the opposing faces (as measured in the z direction) decreases towards the air outlet hole 280 (i.e. as the y axis increases). This slope is relatively gentle - approximately 15 degrees with respect to the y axis. This incline helps to provide a natural and comfortable engagement between the faces of the mouthpiece 251 and the lips of a user. In addition, the exit hole 280 is located where the two mouthpiece faces 251 are closest together, which makes the mouthpiece 250 easy to insert into a user's mouth. In other implementations, different slope angles may be used. For example, instead of 15 degrees with respect to the y axis, a shallower or steeper incline may be used. For example, in some examples the angle of inclination may be in the range 0 to 30 degrees, 5 to 25 degrees or 10 to 20 degrees. It will also be appreciated that the opposing faces may have different angles of inclination. For example, only one of the faces may be inclined with respect to the y axis, or one of the faces may be inclined by a greater amount with respect to the y axis than the other. As can be seen in Figure 3, the front and back faces 251 do not converge completely at the top of the mouthpiece, but rather overhang to provide a small valley or channel 284 which extends in the x-direction of the device. The opening 280, which allows air and vapour to exit from the cartomiser 200, is formed in the centre of this valley 284. Having this small overhang, so that the mouthpiece opening 280 is located in the groove or valley 284, helps to protect the mouthpiece opening from physical contact, and hence from potential damage and dirt (without involving a cap or other form of cover which might be easily lost).

Typically the span of the channel, as measured parallel to the z axis, is in the range 2-10mm, and preferably within the range 3-6mm. This relatively narrow depth helps the overhang of the mouthpiece faces 251 to protect the opening 280, and is also compatible with the generally planar configuration of the cartomiser (i.e. the smaller sizing in the z direction). Typically, the channel has an approximately U-shaped profile, with the depth or indentation of the valley 284 below the overhang (as measured in the y direction) being approximately equal to the span of the valley 284 as given above. This level of indentation allows the overhand of the opposing mouthpiece faces 251 to provide reasonable protection for the exit hole 280.

Typically the valley 284 extends for a distance of at least 6mm, and preferably at least 10mm in the width direction. Note that the exit hole 280, which lies in the middle of the valley, usually halfway along, has a generally circular shape. The size of the exit hole 280 is therefore primarily constrained by the span of the channel, rather than its extension in the width direction, and hence the exit hole 280 may occupy only a relatively small portion of the overall valley 284. It will be appreciated that having the channel extend in the width direction reflects the shaping of the mouthpiece faces 251, which also have their greatest extent in the width direction.

As shown in the central view of Figure 3, the mouthpiece faces 251 have a curved perimeter, i.e. without any corners. This curved perimeter includes a continuously curved section adjacent the exit hole of the mouthpiece, where the curvature lies substantially within the plane defined by the longitudinal direction and the width direction. This curved section again reflects the relative wide sizing of the cartomiser 200, and also means that the device is less likely to snag, e.g. when being removed from a pocket or bag.

Typically, the curved section is substantially parallel to the width direction adjacent the exit hole 280 of the mouthpiece, and the curvature extends for an angle of at least 30 degrees, and preferably at least 45 degrees, around towards the length direction on either side of the exit hole. In other words, the curved section represents a total rotation of at least 60 or 90 degrees, which as shown in Figure 3 will usually be split equally on each side of the mouthpiece hole 280. The radius of curvature of the curved section typically varies around the mouthpiece face 251, but generally is at least 8mm, and preferably at least 12mm, to provide an overall profile which is smooth and without sharp corners.

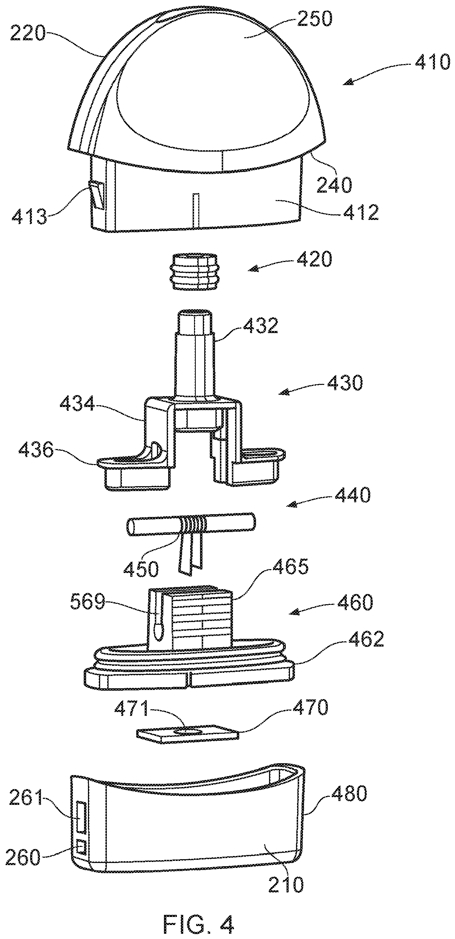

Figure 4 is an exploded view of the cartomiser 200 of the e-cigarette of Figure 1 in accordance with some embodiments of the disclosure. The cartomiser includes a shell 410, a vent seal 420, an inner frame 430, a heating coil 450 located on a wick 440, a primary seal 460 (also referred to as the cartomiser plug), a printed circuit board (PCB) 470 and an end cap 480. The view of Figure 4 shows the above components exploded along the longitudinal (height or y) axis of the cartomiser 200.

The cap 480 is formed from substantially rigid plastic such as polypropylene and provides the base portion 210 of the cartomiser. The cap is provided with two holes 260, 261 on each side (only one side is visible in Figure 4, but the side which is not visible is the same as the side that is visible). The lower hole 260 is for latching the cartomiser 200 to the control unit 300, while the upper hole 261 is for latching the end cap 480 to the shell 410. As described in more detail below, latching the cap 480 and the shell 410 in effect completes the assembly of the cartomiser, and retains the various components shown in Figure 4 in the correct position.

Above the end cap is located the PCB 470, which includes a central air hole 471 to allow air to flow through the PCB into the atomiser (the end cap 480 is likewise provided with a central air hole, not visible in Figure 4) to support this air flow into the atomiser. In accordance with some embodiments, the PCB does not contain any active electrical components, but rather provides a circuit or conductive path between the control unit 300 and the heater 450.

Above the PCB 470 is located the primary seal 460, which has two main portions, an upper portion which defines (in part) an atomizer chamber 465, and a lower portion 462 which acts as an end seal for the reservoir 270. Note that in the assembled cartomiser 200, the reservoir of e-liquid is located around the outside of the atomizer chamber, and the e-liquid is prevented from leaving the cartomiser (at least in part) by the lower portion 462 of the cartomiser plug 460. The cartomiser plug is made from a material that is slightly deformable. This allows the lower portion 462 to be compressed a little when inserted into the shell 410, and hence provide a good seal to retain the e-liquid in reservoir 270.

Two opposing side walls of the atomiser chamber 465 are provided with respective slots 569 into which the wick 440 is inserted. This configuration thereby ensures that the heater 450, which is positioned on the wick, is located near the bottom of the atomiser chamber to vaporise liquid introduced into the atomiser chamber 465 by wick 440. In some embodiments, the wick 440 is made of glass fibre rope (i.e. filaments or strands of glass fibre twisted together), and the heater coil 450 is made of nichrome (an alloy of nickel and chromium). However, various other types of wick and heater are known and could be used in the cartomiser 200, such as a wick made out of porous ceramic, and/or some form of planar heater (rather than a coil). Note that although Figure 4 suggests that the heater coil 450 has a loop of wire dropping down from the wick at each end, in practice there is just a single lead at each end (as described in more detail below).

The cartomiser plug 460 and the wick/heater assembly are surmounted by the inner frame 430, which has three main sections. The inner frame is substantially rigid, and may be made of a material such as polybutylene terephthalate. The lowermost section 436 of the inner frame 430 covers the lower portion 462 of the cartomiser plug 460, while the middle section 434 completes the atomiser chamber 465 of the cartomiser plug. In particular, the inner frame provides the top wall of the atomiser chamber, and also two side walls that overlap with the two side walls of the atomising chamber 465 of the cartomiser plug. The final section of the inner frame is an airflow tube 432 that leads upwards from the top wall of the atomising chamber (part of the middle section 434) and connects with the mouthpiece hole 280. In other words, tube 432 provides a passage for vapour produced in the atomising chamber 465 to be drawn out of the e-cigarette 100 and inhaled through mouthpiece 250.

Since the inner frame is substantially rigid, the vent seal 420 is provided at (inserted around) the top of the airflow tube 432 to ensure a proper seal between the inner frame and the mouthpiece exit hole 280. The vent seal 420 is made of a suitably deformable and resilient material such as silicone. Lastly, the shell 410 provides the external surface of the upper portion 220 of the cartomiser 200, including the mouthpiece 250, and also the lip or flange 240. The shell 410, like the end cap, is formed of a substantially rigid material, such as polypropylene. The lower section 412 of the shell 410 (i.e. below the lip 240) sits inside the end cap 480 when the cartomiser has been assembled. The shell is provided with a latch tab 413 on each side to engage with hole 261 on each side of the end cap 480, thereby retaining the cartomiser 200 in its assembled condition.

The airflow passage through the cartomiser enters a central hole in the cap 480 (not visible in Figure 4) and then passes through a hole 471 in the PCB. The airflow next passes up into the atomiser chamber 465, which is formed as part of the cartomiser plug 460, flows around the wick and heater assembly 500 and through the tube 432 of the inner frame 430 (and through vent seal 420), and finally exits through the hole 280 in the mouthpiece 250.

The reservoir 270 of e-liquid is contained in the space between this airflow passage and the outer surface of the cartomiser 200. Thus shell 410 provides the outer walls (and top) of the housing for the reservoir 270, while the lower section 436 of the inner frame in conjunction with the base portion 462 of the primary seal 460 and end cap 480 provide the bottom or floor of the housing for the reservoir of e-liquid. The inner walls of this housing are provided by the atomising chamber 465 of the primary seal 460, in cooperation with the middle section 434 of the inner frame, and also the airflow tube 432 of the inner frame 430 and the vent seal 420. In other words, the e-liquid is stored in the reservoir space between the outer walls and the inner walls. However, the e-liquid should not penetrate inside the inner walls, into the airflow passage, except via wick 440, otherwise there is a risk that liquid would leak out of the mouthpiece hole 280.

The capacity of this space is typically of the order of 2ml in accordance with some embodiments, although it will be appreciated that this capacity will vary according to the particular features of any given design. Note that unlike for some e-cigarettes, the e-liquid reservoir 270 is not provided with any absorbent material (such as cotton, sponge, foam, etc) for holding the e-liquid. Rather, the reservoir chamber only contains the liquid, so that the liquid can move freely around the reservoir 270. This has certain advantages, such as generally supporting a larger capacity, and also making the filling procedure less complex. One potential disadvantage with having a free liquid in the reservoir (i.e. not holding the liquid in a sponge or other absorbent structure) is that the liquid can flow more easily, and hence might be more likely to leak in an undesirable manner from the reservoir 270 into the airflow passage. However, such leakage is generally prevented by the vent seal 420 and the primary seal 460.

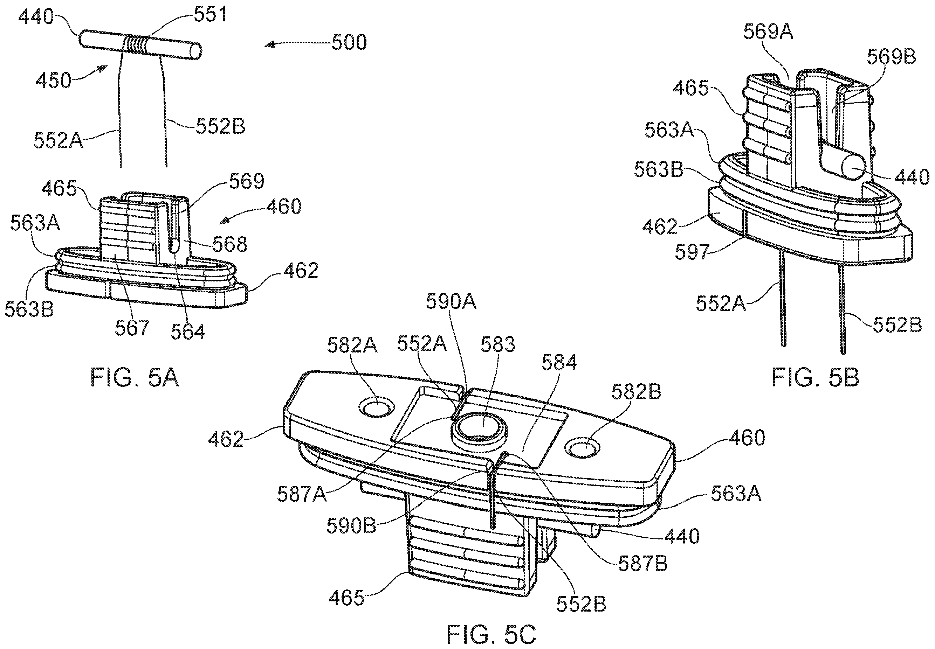

Figure 5A, 5B and 5C illustrate the wick/heater assembly being fitted into the cartomiser plug in accordance with some embodiments of the disclosure. The wick/heater assembly 500 is formed from the heater wire 450 and the wick 440. As noted above, the wick comprises glass fibres formed into a generally cylindrical or rod shape. The heater 450 comprises a coil of wire 551 wound around the wick. At each end of the coil there is a contact wire 552A, 552B, which together act as the positive and negative terminals to allow the coil to receive electrical power.

As visible in Figure 5A, the primary seal 460 includes the base portion 462 and the atomising chamber 465. The base portion is provided with two outwardly directed ribs. When the shell 410 is fitted over the base portion, these ribs are compressed slightly in order to fit inside the shell 410. This compression and the resulting slight resilient deformation of the ribs helps to ensure a good seal for the e-liquid at the base of the cartomiser reservoir.

Also visible in Figure 5A, the atomising chamber 465 comprises four walls in a rectangular arrangement, a pair of opposing side walls 568, and a pair of opposing front and back walls 567. Each of the opposing side walls 568 includes a slot 569 which has an open end at the top (and in the centre) of the side wall, and a closed end 564 relatively near the bottom of the atomising chamber 465 - i.e. the two slots 569 extend more than halfway down their respective side walls 568.

Referring now to Figure 5B, this shows the wick/heater assembly 500 now fitted into the atomising chamber 465 of the cartomiser plug. In particular, the wick/heater assembly is positioned so that it extends between, and protrudes out of, the two opposing slots 569A, 569B. The wick is then lowered until it reaches the closed end 564 of each slot. Note that in this position, the coil 551 is located entirely in the atomizing chamber 465 - it is only the wick itself 440 that extends out of the slots into the reservoir area 270. It will be appreciated that this arrangement allows the wick to draw e-liquid from the reservoir 270 into the atomizing chamber 465 for vaporisation by the wire heater coil 551. Having the wick located near the bottom of the atomizing chamber, and more particularly also near the bottom of the reservoir 270, helps to ensure that the wick retains access to liquid in the reservoir even as the e-liquid is consumed, and hence the level of the e-liquid in the reservoir drops. Figure 5B also shows the heater contact wires 552A, 552B extending below the primary seal 460.

Figure 5C illustrates the underside of the base portion 462 of the primary seal 460. This view shows that the base portion includes two holes 582A, 582B, which are used for filing the reservoir 270 with e-liquid, as described in more detail below. The underside further includes a rectangular indentation 584 for receiving the PCB 470. A central hole 583 is provided in this indentation 584 to provide an air passage from underneath (and outside) the cartomiser into the atomisation (vaporisation) chamber 465. It will be appreciated that after assembly, this central hole 583 in the cartomiser plug is aligned with the corresponding central hole 471 in the PCB.

There are also two much smaller holes 587A, 587B formed in the rectangular indentation 584 of the lower portion of the cartomiser plug 460, one on either side of the central hole 583. The contact wires 552A and 552B extend downwards from the heater 450 and pass respectively through these two holes, 587A, 587B, in order to exit the vaporising chamber 465.

A slit 590A, 590B is formed in each of the front and back walls of the rectangular indentation 584. After extending through the two holes 587A, 587B, each contact wire from the heater is bent flat onto the underside of the cartimoser plug, and then leaves the rectangular indentation via the respective slits 590A, 590B. Thus contact wire 552A passes out of the atomising chamber 465 through hole 587A, and then exits the rectangular indentation 584 via slot 590A; likewise, contact wire 552B passes out of the atomising chamber 465 through hole 587B, and then exits the rectangular indentation 584 via slot 590B. The remaining portion of each wire 552A, 552B is then bent upwards towards the atomising chamber 465 in order to sit within a respective groove 597 in the cartomiser plug 460 (see Figure 5B). In some examples there may not be respective grooves 597 in the cartomiser plug 460 and the remaining portions of the each wire 552A, 552B may instead be simply bent to run alongside the side of cartomiser plug 460.

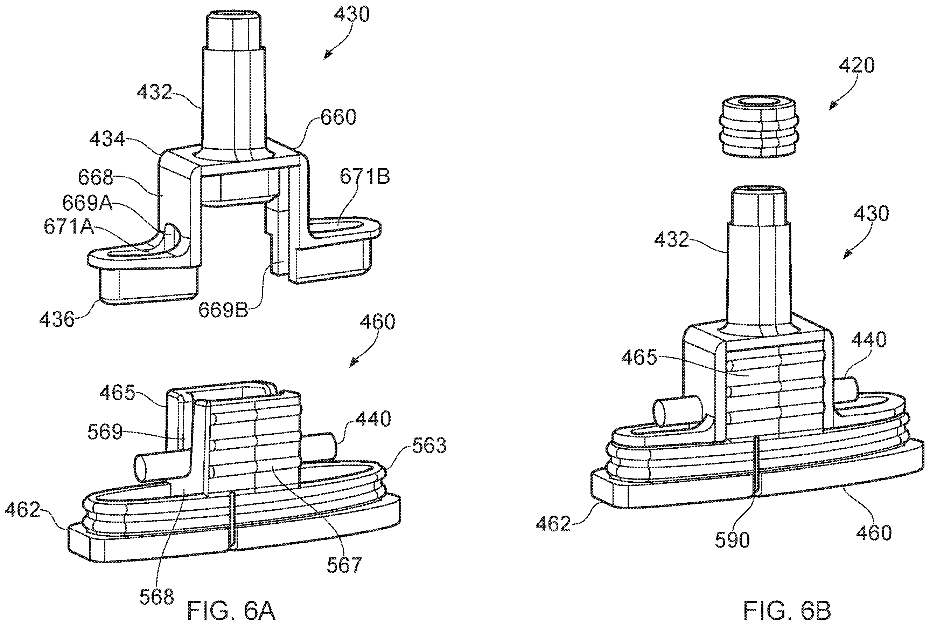

Figures 6A and 6B illustrate the inner frame and the vent seal being fitted into the cartomiser plug in accordance with some embodiments of the disclosure. Thus as previously described, the inner frame 430 comprises a base section 436, a middle section 434 and air tube 432 located at the top of the inner frame. The base section contains two slots 671A, 671B extending in a horizontal sideways direction (parallel to the x axis). As the base section 436 of the inner frame is lowered down past the atomizing chamber 465, the portions of the wick 440 that extend out from each side of the atomizing chamber 465 pass through these slots 671A, 671 B, thereby allowing the base section of the inner frame to be lowered further until it is received in the lower portion 462 of the cartomiser plug.

As noted above, the middle section 434 of the inner frame complements and completes the atomizing chamber 465 of the cartomiser plug 460. In particular, the middle section provides two opposing side walls 668 and a top wall or roof 660. The latter closes the top of the atomizing chamber 465, except in respect of the air tube 432 which extends up from the atomizing chamber 465 to the exit hole 280 of the mouthpiece 250.

Each of the opposing side walls 668 includes a slot 669A, 669B which extends upwards (parallel to the y axis) from the bottom of the side wall to the closed end of the respective slot. Accordingly, as the base section 436 of the inner frame is lowered down past the atomizing chamber 465, the portions of the wick 440 that extend out from each side of the atomizing chamber 465 pass through these slots 669A, 669B (in addition to slots 671A, 671B). This therefore allows the side walls 668 of the inner frame 430 to overlap the side walls 568 of the cartomiser plug. Further downward movement of the inner frame 430 is prevented once the closed end of slots 669A, 669B contacts the wick 440, which coincides with the base section 436 of the inner frame being received into the lower portion 462 of the cartomiser plug. At this stage, the combination of cartomiser plug 460, heater/wick assembly 500, and inner frame 430, as shown in Figure 6B has been formed, and the vent seal 420 can now be fitted onto the air tube (pipe) 432 of the inner frame 430.

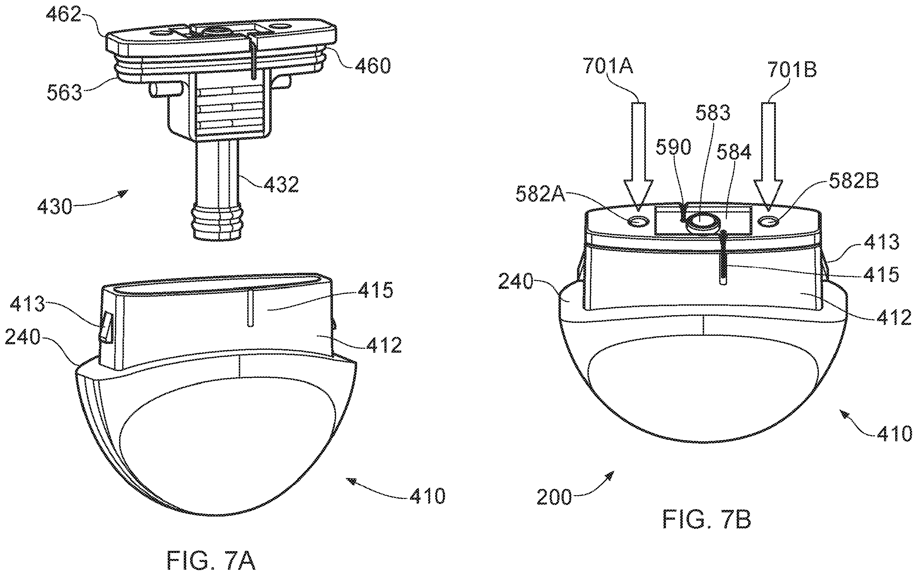

Figure 7A illustrates the combination of the inner frame 430, wick/heater assembly 500, and primary seal 460 being fitted into the shell 410. As this insertion occurs, the slot 415 in each of the front and back faces of the lower portion 412 of the shell 410 accommodates a portion of wire 552 that has passed through slot 590 and has been wrapped back up around the outside of the cartomiser plug 460 and into groove 597. Furthermore, the deformable ribs 563 around the lower portion 462 of the primary seal are slightly compressed by the inside wall of the lower portion 412 of the shell 410 during the insertion, and thereby form a seal to retain the e-liquid in the resulting reservoir 270. Accordingly, as illustrated in Figure 7B, the cartomiser 200 is now ready for filling with the e-liquid. This filling is performed, as indicated by arrows 701A, 701B, through holes 582A and 582B in the primary seal 460, and through slots 671A, 671 B in the inner frame (not visible in Figure 7B).

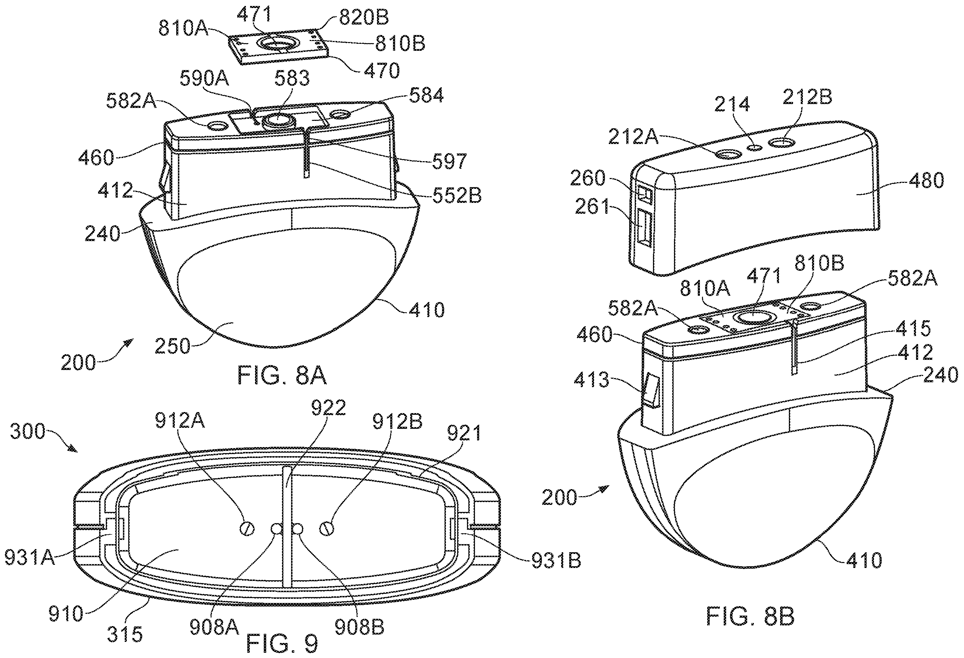

Figure 8A illustrates the PCB 470 being fitted into the rectangular indentation 584 in the underside of the primary seal 460. This fitting aligns the central hole 471 in the PCB with the central hole 583 in the primary seal 460 in order to provide the main airflow channel into the cartomiser 200.

As previously described, the rectangular indentation 584 is provided with a pair of holes 587, located on either side of the central hole 583. Each hole allows egress of a respective contact wire 552A, 552B from the vaporiser chamber 465. The contact wires 552A, 552B are bent flat against the floor of the rectangular indentation 584, and then exit the rectangular indentation 584 via respective slots 590A, 590B in the front and back walls of the rectangular indentation. The final portion of each heater contact wire 552A, 552B, is then bent upwards, back towards the top of the cartomiser and mouthpiece 250, and located in a corresponding groove or channel 597 formed in the cartomiser plug. In addition, the base portion of the shell also includes a slot 415 on each of the front and back faces to accommodate a respective heater contact wire 552A, 552B.

In accordance with some embodiments, the PCB 470 does not contain any active components, but rather provides two large contact pads 810A, 810B on either side of the central hole 471. These contact pads are visible in Figure 8A on the lower face of the PCB, i.e. the side facing the control unit 300 after assembly. The opposite face of the PCB, i.e. the upper side which is received into the rectangular indentation 584 and faces the heater 450, is provided with a similar, corresponding configuration of contact pads (not visible in Figure 8A). The heater contact wires 552A, 552B are in physical, and hence electrical, contact with a respective contact pad on the upper side of the PCB.

The opposing pairs of contact pads on either side of the PCB 470 are connected by respective sets of one or more vias 820A, 820B. In other words, vias 820A provide a conductive path between one contact pad on the lower face of the PCB and a corresponding contact pad on the upper face of the PCB, and vias 820B provide a conductive path between the other contact pad on the lower face of the PCB and its corresponding contact pad on the upper face of the PCB. Accordingly, when the control unit is connected to the cartomiser, pins from the control unit touch the contact pads on the lower side of the PCB 470, and electrical current flows to/from the heater 450 through the respective vias, contact pads on the upper side of the PCB 470, and respective heater contact wires 552A, 552B.

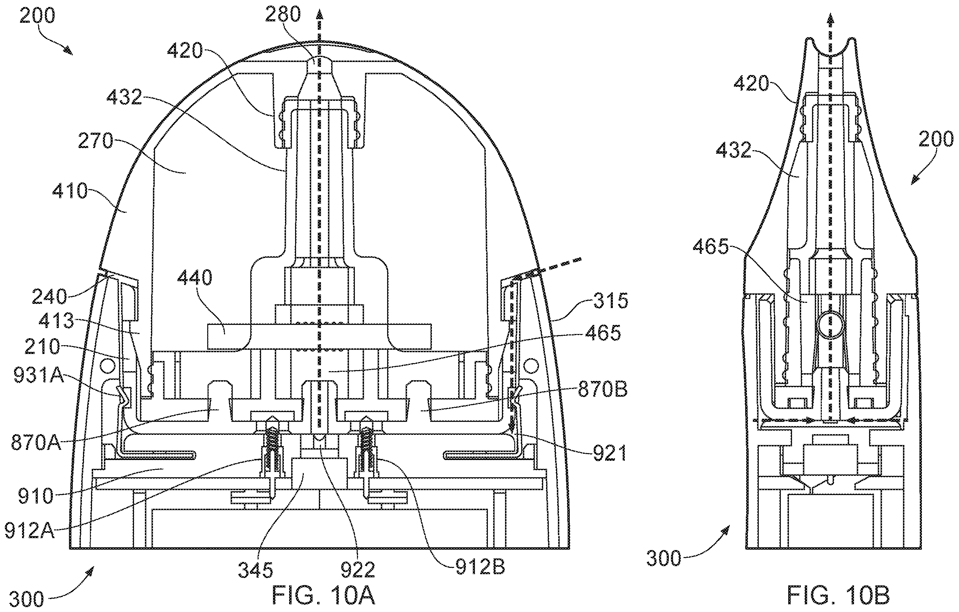

Figure 8B illustrates the end cap 480 being fitted to the cartomiser 200 in accordance with some embodiments of the disclosure. In particular, the end cap 480 is fitted over the end of the cartomiser plug 460 and the lower section 412 of the shell 410, and is retained in this position by the protruding member 413 provided on each side of the lower section 412 of the shell engaging into the corresponding hole or slot 261 on each side of the end cap. In this fully assembled state (see Figure 2), the end cap 480 covers and therefore closes the holes 582A, 582B in the cartomiser plug that were used for filling the liquid reservoir 270. Indeed, as can be seen in Figure 10A, the end cap 480 is provided with two upwardly directed plugs 870A and 870B that respectively penetrate and close the filling holes 582A, 582B. Accordingly, the reservoir 270 is now fully sealed, apart from the opening on each side of the atomising chamber 465 through which the wick 440 passes into the atomising chamber 465.

As previously discussed, the end cap includes three holes, a central hole 214 and two holes 212A, 212B located on either side of this central hole. The fitting of the end cap 480 aligns the central hole 214 of the end cap with the central hole 471 in the PCB and with the central hole 583 in the primary seal 460 in order to provide the main airflow channel into the cartomiser 200. The two side holes 212A, 212B allow pins from the control unit 300, acting as positive and negative terminals, to pass through the end cap 480 and make contact with respective contact pads 810A, 810B on the lower side of the PCB, thereby enabling the battery 350 in the control unit 300 to supply power to the heater 450.

In accordance with some embodiments, the primary seal 460, which as noted above is made of a resilient deformable material such as silicone, is held in a compressed state between the inner frame 430 and the end cap 480. In other words, the end cap is pushed onto the cartomiser 200 and compresses the primary seal 460 slightly before the latch components 413 and 261 engage with one another. Consequently, the primary seal remains in this slightly compressed state after the end cap 480 and shell 410 are latched together. One advantage of this compression is that the end cap acts to push the PCB 470 onto the heater contact wires 552A, 550B, thereby helping to ensure a good electrical connection without the use of solder.

Figure 9 is a top view looking down onto the control unit 300 of the e-cigarette of Figure 1 in accordance with some embodiments of the disclosure. The control unit includes external walls 315 that rise above the rest of the control unit (as best seen in Figure 1) to define a cavity for accommodating the lower portion 210 of the cartomiser. Each side of these walls 315 is provided with a spring clip 931A, 931 B that engages with the hole or slot 260 on each side of the cartomiser 200 (see Figure 2), thereby retaining the cartomiser in engagement with the control unit 300 to form the assembled e-cigarette 100.

At the bottom of the cavity formed by the upper portion of control unit walls 315 (but otherwise at the top of the main body of the control unit 300) is a battery seal 910 (see also Figure 1). The battery seal 910 is formed from a resilient (and compressible) material such as silicone. The battery seal 910 helps to mitigate one potential risk with an e-cigarette 100, which is that e-liquid leaks from the reservoir 270 into the main air passage through the device (this risk is greater where there is free liquid in the reservoir, rather than the liquid being held by a foam or other such material). In particular, if e-liquid were able to leak into the portion of the control unit containing the battery 350 and control electronics, then this might short circuit or corrode such components. Furthermore, there is also a risk that the e-liquid itself would then become contaminated before returning into the cartomiser 200 and then exiting through the mouthpiece hole 280. Accordingly, if any e-liquid does leak into the central air passage of the cartomiser, the battery seal 910 helps to prevent such leakage progressing into the portion of the control unit that contains the battery 350 and control electronics. (The small holes 908 in the battery seal 910 do provide very limited fluid communication with the microphone 345 or other sensor device, but the microphone 345 itself can then act as a barrier against any such leakage progressing further into the control unit.

As shown in Figure 9, there is a small groove or spacing 921 around the perimeter between the top of the battery seal 910 and the inside of the walls 315 of the control unit; this is primarily formed by the rounded corner of the battery seal 910. The battery seal is further provided with a central groove 922 from front to back, which connects at both ends (front and back) with the perimeter groove 921 to support airflow into the cartomiser, as described in more detail below. Immediately adjacent to central groove 922 are two holes 908A, 908B, one on either side of the groove 922. These air holes extend down to the microphone 345. Thus when a user inhales, this causes a drop in pressure within the central air passage through the cartomiser 200, as defined by air tube 432, the central hole 583 in the primary seal 460, etc, and also within the central groove 922, which lies at the end of this central air passage. The drop in pressure further extends through holes 908A, 908B to the microphone 345, which detects the drop in pressure, and this detection is then used to trigger activation of the heater 450.

Also shown in Figure 9 are two contact pins, 912A, 912B, which are linked to the positive and negative terminals of the battery 350. These contact pins 912A, 912B pass through respective holes in the battery seal 910 and extend through holes 212A, 212B of the end cap to make contact with contact pads 810A, 810B respectively on the PCB. Accordingly, this then provides an electrical circuit for supplying electrical power to the heater 450. The contact pins may be resiliently mounted within the battery seal (sometimes referred to as "pogo pins"), such that the mounting is under compression when the cartomiser 200 is latched to the control unit 300. This compression causes the mounting to press the contact pins against the PCB contact pads 810A, 810B, thereby helping to ensure good electrical connectivity. It will be appreciated that approaches other than using pogo pins could be used. For example, in some cases the contact pins may not be spring mounted, but may instead accommodate a degree of resilient deflection when assembled to facilitate a biased contract with the PCB contact pads. In another cases, the contact pins may themselves be rigid and carried by a resiliently mounted support.

The battery seal 910, which as noted above is made of a resilient deformable material such as silicone, is held in a compressed state between the cartomiser 200 and the control unit 300. In other words, inserting the cartomiser into the cavity formed by walls 315 causes the end cap 480 of the cartomiser to compress the battery seal 910 slightly before the spring clips 931A, 931B of the control unit engage with the corresponding holes 260A, 260B in the lower portion 210 of the cartomiser. Consequently, the battery seal 910 remains in this slightly compressed state after the cartomiser 200 and the control unit 300 are latched together, which helps to provide protection against any leakage of e-liquid, as discussed above.

Figures 10A and 10B are cross-sections respectively (a) from side to side, and (b) from front to back, showing the airflow through the e-cigarette of Figure 1 in accordance with some embodiments of the disclosure. The airflow is denoted in Figures 10A and 10B by the heavy black, dashed arrows. (Note that Figure 10A only shows air flow on one side of the device, but there is an analogous air flow on the other side as well - having multiple such air inlets reduces the risk that a user will accidentally block the air inlets with their fingers while holding the device).

The airflow enters through a gap at the sides of the e-cigarette 100, in between the top of the walls 315 of the control unit, and the flange or rim 240 of the cartomiser shell 410. The airflow then passes down a slight spacing between the inside of the walls 315 and the outside of the lower portion 210 of the cartomiser 200, past the spring clips 931, and hence into perimeter groove 921 (as shown in Figure 9). The airflow is then drawn around the perimeter groove 921, and hence out of the plane of Figures 10A and 10B (so that this portion of the airflow path is therefore not visible in these two diagrams). Note that there is typically some space above the groove 921 between the inside of the control unit walls and the outside of the cartomiser end cap, so the airflow is not necessarily constrained to the groove 921 per se.

After travelling an angle of approximately 90 degrees around the perimeter groove 921, the airflow passes into the central groove 922, from where it travels to and through the central hole 583 of the end cap 480 and hence into the central air passage of the cartomiser. Note that Figure 10B shows this airflow along the central groove 922 into the central air passage, and then the flow of air up through the central air passage is shown in both Figures 10A and 10B. In contrast to groove 921, the space above groove 922 is not open, but rather the battery seal 910 is compressed against the end cap 480 of the cartomiser 200. This configuration results in the end cap covering the groove to form a closed channel having a confined space. This confined channel can be utilised to help control the draw resistance of the e-cigarette 100, as described in more detail below.

There are various benefits associated with the overall airflow path such as shown in Figures 10A and 10B. The airflow detector, such as microphone 345, is generally located in the control unit 300. This reduces cost because the microphone is therefore in the reusable portion of the device, and so there is no need to include a microphone in every cartomiser (the disposable component). In addition, having the microphone 345 in the control unit 300 allows the microphone to be readily connected to the battery 350 and to the control processor of the control unit (not shown in the Figures).

On the other hand, it is generally desirable to reduce or avoid an airflow past electronics components, for example, because such electronics components tend to become warm with use, and may potentially shed volatiles. It will be appreciated that the airflow path shown in Figures 10A and 10B largely bypasses the electronic components of the control unit, with only the small holes 908 branching off this main airflow to allow the microphone 345 to detect a change in pressure. This avoidance of airflow past the main electronic components of the control unit has been achieved despite the fact that the cartomizer sits quite deeply within the control unit (which helps to reduce the overall length of the device).

Furthermore, in many existing e-cigarettes, the overall air path is not tightly controlled. For example, air may leak into the air path at joins between various components (such as between the cartomiser and control unit), rather than just at the dedicated air inlet(s). This leakage (as well as various other manufacturing variations) may result in significant variation in the draw resistance of the device, where the draw resistance in effect represents the pressure difference needed to produce a given air flow through the device. This variation in draw resistance can prevent a consistent user experience and can also effect the operation of the device. For example, if the draw resistance is high, it is likely that the flow of air through the device may be reduced, which in turn reduces the amount of air cooling experienced by the heater.

Accordingly, the approach described herein provides an e-cigarette device including: an atomiser for vaporising a liquid; an air passage through the atomiser, the air passage exiting the e-cigarette at a mouthpiece; at least one air inlet joined by a channel to the air passage through the vaporiser; and at least one resilient seal which acts to prevent air from the air inlet travelling to the air passage except through the channel.

For example, in the implementation described above, the air flow entering the central air passage through the vaporiser must first travel along groove 922. This groove, in conjunction with the bottom of the end cap 480 that in effect provides a top surface or closure for the groove, defines the airflow channel through the control unit into the cartomiser.

In such a device, air from the air inlet must necessarily travel through the channel to reach the air passage (because the seal prevents other routes). Accordingly, the channel provides a point of control for the draw resistance - especially if the channel provides the majority of the draw resistance for the air path through the whole device. In particular, as long as the draw resistance for the channel (which is determined largely by the size of the channel) is reasonably constant between devices (and between different usages of the same device), then the draw resistance for the device as a whole will likewise be reasonably constant.

In some implementations, the e-cigarette may further comprise a facility to alter the predetermined draw resistance for the e-cigarette. This facility may allow a user to set the predetermined draw resistance for the e-cigarette to one of a limited number of discrete values according to individual preference, etc. For example, for the e-cigarette described herein, there may be two successive latch positions between the cartomiser 200 and the control unit 300, which result in a lower or greater compression of the battery seal 910. The lower compression will generally allow groove 922 to expand slightly, and hence provide a lower draw resistance than the latch position which produces the higher compression of the battery seal. Another way of implementing this facility would be to provide some baffle that can be moved into the channel or groove 922 to partly obstruct the airflow by a desired amount.

The seal may be formed of a resilient material, such as silicone, and the channel is formed at least in part by the seal material itself. For example, in some embodiments, the channel is defined by a resilient material compressed against a surface of a rigid material, such as the battery seal 910 pressing against the end cap 480, and the surface of the rigid material may include a hole, such as hole 583 in end cap 480, that connects from the channel 922 into the air passage through the atomiser. Note that the channel may in fact comprises a network of multiple (sub)channels as appropriate, according to the particular implementation.

As described above, the device may include a cartomiser 200 and a control unit 300, and the resilient seal is provided as part of the control unit that contacts the exterior of the cartomiser when the cartomiser is joined to the control unit. The resilient material may be held under compression between the cartomiser and the control unit when the cartomiser is joined to the control unit, such as by a latch mechanism. This compression of the resilient material helps to provide an air-tight seal around the edges of the seal.

A further consideration is that for some e-cigarettes, there is a risk that the e-liquid may leak 270 into main air passage. In such a situation, the seal helps to ensure that the e-liquid is only able to travel from the air passage into the air channel, thereby helping to prevent the e-liquid coming into contact with the battery and other electrical components. Furthermore, the air channel may be sufficiently narrow to prevent significant flow of e-liquid through the channel, which further helps to constrain any leaked e-liquid.

Although various embodiments have been described in detail herein, this is by way of example only, it will be appreciated that a channel to constrain airflow into a device may be utilised in many different configurations. For example, this approach might be used for a one-piece or three-piece device (rather than a two-piece device, i.e. cartomiser and control unit, as described here). Similarly, this approach could be utilised with electronic vapour provision systems that includes material derived from tobacco plants which is provided in any suitable form (powder, paste, shredded leaf material, etc, i.e. not liquid), and then heated to produce volatiles for inhalation by a user. This approach could also be used with various types of heater for the e-cigarette, various types of airflow configuration, various types of connection between the cartomiser and the control unit (such as screw or bayonet) etc. The skilled person will be aware of various other forms of electronic vapour provision system which might utilise a channel for restricting the airflow as described herein.

Furthermore, it will be appreciated the manner of cartomiser assembly set out above is merely one example, and an assembly process comprising different steps, or a similar steps performed in a different order may also be adopted. For example, with reference to the steps set out in relation to Figures 6B, 7A and 7B, in another example instead of fitting the vent seal 420 to the air tube (pipe) 432 of the inner frame (Figure 6B) before placing the combined assembly in the shell 410 (Figures 7A and 7B), the vent seal 420 might first be mounted in position in the shell 410 so that it mounts to the air tube (pipe) 432 of the inner frame when the inner frame 430, wick/heater assembly 500, and primary seal 460 are together fitted into the shell 410. Similarly, with reference to the steps set out in relation to Figures 8A and 8B, in another example instead of placing the PCB 470 in its indentation 584 in the cartomiser plug 460 before attaching the cap 480 to complete the cartomiser assembly, the PCB 470 might first be mounted in position in the cap 480, and then the cap 480, with PCB 470 attached, connected to the shell 410. The PCB 470 may mount to the cap 480 by a friction / press fit, for example. The cap may include locating pegs, or other guide mechanism, to help position the PCB in the cap so it is aligned with the indentation 584 in the cartomiser plug when the cap is attached to the shell.

In conclusion, in order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope of the claims. Various embodiments may suitably comprise, consist of, or consist essentially of, various combinations of the disclosed elements, components, features, parts, steps, means, etc other than those specifically described herein.

## Claims

1. A cartridge (200) for a vapour provision device (100), the cartridge including a mouthpiece (250) having a length direction corresponding to the direction of insertion of the vapour provision device into the mouth of a user, a width direction corresponding to the direction along a lip-line of the user, and a depth direction corresponding to the opening direction of lips of the user, the mouthpiece comprising first and second opposing faces (251) and an exit hole (280) located between the first and second opposing faces, wherein the opposing faces are approximately planar and inclined towards one another with respect to the length direction, **characterised in that** the first and second opposing faces of the mouthpiece form outer walls of a reservoir (270) for a liquid within the cartridge.

2. A vapour provision device (100) including a mouthpiece (250) having a length direction corresponding to the direction of insertion of the vapour provision device into the mouth of a user, a width direction corresponding to the direction along a lip-line of the user, and a depth direction corresponding to the opening direction of lips of the user, the mouthpiece comprising first and second opposing faces (251) and an exit hole (280) located between the first and second opposing faces, wherein the opposing faces are approximately planar and inclined towards one another with respect to the length direction, **characterised in that** the first and second opposing faces of the mouthpiece form outer walls of a reservoir (270) for a liquid within the vapour provision device.

3. The cartridge of claim 1 or the vapour provision device of claim 2, wherein each of the opposing faces has a width which is greater than its length.

4. The cartridge of claim 1 or 3 or the vapour provision device of claim 2 or 3, wherein the width of the opposing faces is at least 20mm, and preferably at least 25mm.

5. The cartridge of claim 1 or any claim dependent thereon, or the vapour provision device of claim 2 or any claim dependent thereon, wherein the length of the opposing faces is within the range 8-24mm and preferably within the range 12-20mm.

6. The cartridge of claim 1 or any claim dependent thereon, or the vapour provision device of claim 2 or any claim dependent thereon, wherein the width of the opposing faces is at least 30%, and preferably at least 50%, greater than the length of the opposing faces.

7. The cartridge of claim 1 or any claim dependent thereon, or the vapour provision device of claim 2 or any claim dependent thereon, wherein each of the opposing faces has a continuously curved section adjacent the exit hole of the mouthpiece, and wherein the curvature lies substantially within the plane defined by the longitudinal direction and the width direction.

8. The cartridge or vapour provision device of claim 7, wherein the curved section is substantially parallel to the width direction adjacent the exit hole of the mouthpiece, and said curvature extends for an angle of at least 30 degrees, and preferably at least 45 degrees, towards the length direction on either side of the exit hole, and/or wherein the radius of curvature of the curved section is at least 8mm, and preferably at least 12mm.

9. The cartridge of claim 1 or any claim dependent thereon, or the vapour provision device of claim 2 or any claim dependent thereon, wherein the mouthpiece further comprises a channel (284) located between the first and second opposing faces, the channel extending in the width direction and having an indentation in the length direction, wherein the exit hole is located in said channel.

10. The cartridge or vapour provision device of claim 9, wherein a span of the channel, as measured in the depth direction, is in the range 2-10mm, and preferably within the range 3-6mm, and/or wherein the channel extends for a distance of at least 6mm, and preferably at least 10mm in the width direction, and/or wherein the channel has an approximately U-shaped profile.

11. The cartridge of claim 1 or any claim dependent thereon, or the vapour provision device of claim 2 or any claim dependent thereon wherein the first and second opposing faces are slightly concave along the width and/or the length direction.

12. The cartridge of claim 1 or any claim dependent thereon, or the vapour provision device of claim 2 or any claim dependent thereon, wherein the first and second opposing faces each have a curved perimeter without corners.

13. The cartridge of claim 1 or any claim dependent thereon, or the vapour provision device of claim 2 or any claim dependent thereon, wherein the first opposing face is located in a first plane and the second opposing face is located in a second plane, and wherein the first and second planes are slightly inclined towards one another with respect to the length direction.

14. The cartridge or vapour provision device of claim 13, wherein the exit hole is located where the first and second opposing faces are closest together.

15. The cartridge of claim 1 or any claim dependent thereon, or the vapour provision device of claim 2 or any claim dependent thereon, wherein the first and second opposing faces are inclined towards one another with respect to the length direction by an angle of inclination in the range 5 to 25 degrees, or in the range 10 to 20 degrees, and preferably about 15 degrees.

## Patentansprüche

1. Patrone (200) für eine Dampfbereitstellungsvorrichtung (100), wobei die Patrone ein Mundstück (250) mit einer Längsrichtung, die der Einführungsrichtung der Dampfbereitstellungsvorrichtung in den Mund eines Benutzers entspricht, einer Breitenrichtung, die der Richtung entlang einer Lippenlinie des Benutzers entspricht, und einer Tiefenrichtung, die der Öffnungsrichtung der Lippen des Benutzers entspricht, aufweist, wobei das Mundstück eine erste und eine zweite gegenüberliegende Fläche (251) und ein zwischen der ersten und der zweiten gegenüberliegenden Fläche angeordnetes Austrittsloch (280) umfasst, wobei die gegenüberliegenden Flächen annähernd eben und in Bezug auf die Längsrichtung zueinander geneigt sind, **dadurch gekennzeichnet, dass** die erste und die zweite gegenüberliegende Fläche des Mundstücks Außenwände eines Vorratsbehälters (270) für eine Flüssigkeit innerhalb der Patrone bilden.

2. Dampfbereitstellungsvorrichtung (100), umfassend ein Mundstück (250) mit einer Längsrichtung, die der Einführungsrichtung der Dampfbereitstellungsvorrichtung in den Mund eines Benutzers entspricht, einer Breitenrichtung, die der Richtung entlang einer Lippenlinie des Benutzers entspricht, und einer Tiefenrichtung, die der Öffnungsrichtung der Lippen des Benutzers entspricht, aufweist, wobei das Mundstück eine erste und eine zweite gegenüberliegende Fläche (251) und ein zwischen der ersten und der zweiten gegenüberliegenden Fläche angeordnetes Austrittsloch (280) umfasst, wobei die gegenüberliegenden Flächen annähernd eben und in Bezug auf die Längsrichtung zueinander geneigt sind, **dadurch gekennzeichnet, dass** die erste und die zweite gegenüberliegende Fläche des Mundstücks Außenwände eines Vorratsbehälters (270) für eine Flüssigkeit innerhalb der Dampfbereitstellungsvorrichtung bilden.

3. Patrone gemäß Anspruch 1 oder Dampfbereitstellungsvorrichtung gemäß Anspruch 2, wobei jede der gegenüberliegenden Flächen eine Breite aufweist, die größer als ihre Länge ist.

4. Patrone gemäß Anspruch 1 oder 3 oder Dampfbereitstellungsvorrichtung gemäß Anspruch 2 oder 3, wobei die Breite der gegenüberliegenden Flächen mindestens 20 mm und vorzugsweise mindestens 25 mm beträgt.

5. Patrone gemäß Anspruch 1 oder gemäß einem davon abhängigen Anspruch oder Dampfbereitstellungsvorrichtung gemäß Anspruch 2 oder gemäß einem davon abhängigen Anspruch, wobei die Länge der gegenüberliegenden Flächen im Bereich von 8-24 mm und vorzugsweise im Bereich von 12-20 mm liegt.

6. Patrone gemäß Anspruch 1 oder gemäß einem davon abhängigen Anspruch oder Dampfbereitstellungsvorrichtung gemäß Anspruch 2 oder gemäß einem davon abhängigen Anspruch, wobei die Breite der gegenüberliegenden Flächen mindestens 30%, vorzugsweise mindestens 50%, größer als die Länge der gegenüberliegenden Flächen ist.

7. Patrone gemäß Anspruch 1 oder gemäß einem davon abhängigen Anspruch oder Dampfbereitstellungsvorrichtung gemäß Anspruch 2 oder gemäß einem davon abhängigen Anspruch, wobei jede der gegenüberliegenden Flächen einen kontinuierlich gekrümmten Abschnitt angrenzend an das Austrittsloch des Mundstücks aufweist und wobei die Krümmung im Wesentlichen innerhalb der durch die Längsrichtung und die Breitenrichtung definierten Ebene liegt.

8. Patrone oder Dampfversorgungsvorrichtung gemäß Anspruch 7, wobei der gekrümmte Abschnitt im Wesentlichen parallel zur Breitenrichtung angrenzend an das Austrittsloch des Mundstücks ist und sich die Krümmung über einen Winkel von mindestens 30 Grad und vorzugsweise mindestens 45 Grad zur Längsrichtung auf beiden Seiten des Austrittslochs erstreckt, und/oder wobei der Krümmungsradius des gekrümmten Abschnitts mindestens 8 mm und vorzugsweise mindestens 12 mm beträgt.

9. Patrone gemäß Anspruch 1 oder gemäß einem davon abhängigen Anspruch, oder Dampfbereitstellungsvorrichtung gemäß Anspruch 2 oder gemäß einem davon abhängigen Anspruch, wobei das Mundstück ferner einen Kanal (284) umfasst, der sich zwischen der ersten und der zweiten gegenüberliegenden Fläche befindet, wobei sich der Kanal in Breitenrichtung erstreckt und eine Einbuchtung in Längsrichtung aufweist, wobei sich das Austrittsloch in dem Kanal befindet.

10. Patrone oder Dampfbereitstellungsvorrichtung gemäß Anspruch 9, wobei eine Spanne des Kanals gemessen in Tiefenrichtung im Bereich von 2-10 mm und vorzugsweise im Bereich von 3-6 mm liegt und/oder wobei sich der Kanal über eine Entfernung von mindestens 6 mm und vorzugsweise mindestens 10 mm in Breitenrichtung erstreckt und/oder wobei der Kanal ein annähernd U-förmiges Profil aufweist.

11. Patrone gemäß Anspruch 1 oder gemäß einem davon abhängigen Anspruch oder Dampfbereitstellungsvorrichtung gemäß Anspruch 2 oder gemäß einem davon abhängigen Anspruch, wobei die erste und die zweite gegenüberliegende Fläche entlang der Breiten- und/oder Längsrichtung leicht konkav sind.

12. Patrone gemäß Anspruch 1 oder gemäß einem davon abhängigen Anspruch oder Dampfbereitstellungsvorrichtung gemäß Anspruch 2 oder gemäß einem davon abhängigen Anspruch, wobei die erste und die zweite gegenüberliegende Fläche jeweils einen gekrümmten Umfang ohne Ecken aufweisen.

13. Patrone gemäß Anspruch 1 oder gemäß einem davon abhängigen Anspruch oder die Dampfbereitstellungsvorrichtung gemäß Anspruch 2 oder gemäß einem davon abhängigen Anspruch, wobei sich die erste gegenüberliegende Fläche in einer ersten Ebene und die zweite gegenüberliegende Fläche in einer zweiten Ebene befindet und wobei die erste und die zweite Ebene in Bezug auf die Längsrichtung leicht zueinander geneigt sind.

14. Patrone oder Dampfbereitstellungsvorrichtung gemäß Anspruch 13, wobei sich das Austrittsloch dort befindet, wo die erste und zweite gegenüberliegende Fläche am nächsten beieinander liegen.

15. Patrone gemäß Anspruch 1 oder gemäß einem davon abhängigen Anspruch oder Dampfbereitstellungsvorrichtung gemäß Anspruch 2 oder gemäß einem davon abhängigen Anspruch, wobei die erste und die zweite gegenüberliegende Fläche in Bezug auf die Längsrichtung um einen Neigungswinkel im Bereich von 5 bis 25 Grad oder im Bereich von 10 bis 20 Grad und vorzugsweise um etwa 15 Grad zueinander geneigt sind.

## Revendications

1. Cartouche (200) destinée à un dispositif (100) de fourniture de vapeur, la cartouche comprenant un embout buccal (250) présentant une direction longitudinale correspondant à la direction d'insertion du dispositif de fourniture de vapeur dans la bouche d'un utilisateur, une direction transversale correspondant à la direction suivant une ligne des lèvres de l'utilisateur, et une direction de profondeur correspondant à la direction d'ouverture des lèvres de l'utilisateur, l'embout buccal comportant des première et seconde faces opposées (251) et un trou (280) de sortie situé entre les première et seconde faces opposées, les faces opposées étant approximativement planes et inclinées l'une vers l'autre par rapport à la direction longitudinale, **caractérisée en ce que** les première et seconde faces opposées de l'embout buccal forment des parois extérieures d'un réservoir (270) destiné à un liquide à l'intérieur de la cartouche.

2. Dispositif (100) de fourniture de vapeur comprenant un embout buccal (250) présentant une direction longitudinale correspondant à la direction d'insertion du dispositif de fourniture de vapeur dans la bouche d'un utilisateur, une direction transversale correspondant à la direction suivant une ligne des lèvres de l'utilisateur, et une direction de profondeur correspondant à la direction d'ouverture des lèvres de l'utilisateur, l'embout buccal comportant des première et seconde faces opposées (251) et un trou (280) de sortie situé entre les première et seconde faces opposées, les faces opposées étant approximativement planes et inclinées l'une vers l'autre par rapport à la direction longitudinale, **caractérisé en ce que** les première et seconde faces opposées de l'embout buccal forment des parois extérieures d'un réservoir (270) destiné à un liquide à l'intérieur du dispositif de fourniture de vapeur.

3. Cartouche selon la revendication 1 ou dispositif de fourniture de vapeur selon la revendication 2, chacune des faces opposées présentant une largeur qui est supérieure à sa longueur.

4. Cartouche selon la revendication 1 ou 3 ou dispositif de fourniture de vapeur selon la revendication 2 ou 3, la largeur des faces opposées étant d'au moins 20 mm, et de préférence d'au moins 25 mm.

5. Cartouche selon la revendication 1 ou toute revendication dépendante de celle-ci, ou dispositif de fourniture de vapeur selon la revendication 2 ou toute revendication dépendante de celle-ci, la longueur des faces opposées se trouvant à l'intérieur de la plage de 8 à 24 mm et de préférence à l'intérieur de la plage de 12 à 20 mm.

6. Cartouche selon la revendication 1 ou toute revendication dépendante de celle-ci, ou dispositif de fourniture de vapeur selon la revendication 2 ou toute revendication dépendante de celle-ci, la largeur des faces opposées étant supérieure d'au moins 30%, et de préférence d'au moins 50%, à la longueur des faces opposées.

7. Cartouche selon la revendication 1 ou toute revendication dépendante de celle-ci, ou dispositif de fourniture de vapeur selon la revendication 2 ou toute revendication dépendante de celle-ci, chacune des faces opposées présentant une section continûment incurvée au voisinage du trou de sortie de l'embout buccal, et la courbure se situant sensiblement dans le plan défini par la direction longitudinale et la direction transversale.

8. Cartouche ou dispositif de fourniture de vapeur selon la revendication 7, la section incurvée étant sensiblement parallèle à la direction transversale au voisinage du trou de sortie de l'embout buccal, et ladite courbure s'étendant sur un angle d'au moins 30 degrés, et de préférence d'au moins 45 degrés, vers la direction longitudinale de part et d'autre du trou de sortie, et/ou le rayon de courbure de la section incurvée étant d'au moins 8 mm, et de préférence d'au moins 12 mm.

9. Cartouche selon la revendication 1 ou toute revendication dépendante de celle-ci, ou dispositif de fourniture de vapeur selon la revendication 2 ou toute revendication dépendante de celle-ci, l'embout buccal comportant en outre un canal (284) situé entre les première et seconde faces opposées, le canal s'étendant dans la direction transversale et présentant une indentation dans la direction longitudinale, le trou de sortie étant situé dans ledit canal.

10. Cartouche ou dispositif de fourniture de vapeur selon la revendication 9, une étendue du canal, telle que mesurée dans la direction de profondeur, se trouvant dans la plage de 2 à 10 mm, et de préférence à l'intérieur de la plage de 3 à 6 mm, et/ou le canal s'étendant sur une distance d'au moins 6 mm, et de préférence d'au moins 10 mm dans la direction transversale, et/ou le canal présentant un profil approximativement en forme de U.

11. Cartouche selon la revendication 1 ou toute revendication dépendante de celle-ci, ou dispositif de fourniture de vapeur selon la revendication 2 ou toute revendication dépendante de celle-ci, les première et seconde faces opposées étant légèrement concaves suivant la direction transversale et/ou longitudinale.

12. Cartouche selon la revendication 1 ou toute revendication dépendante de celle-ci, ou dispositif de fourniture de vapeur selon la revendication 2 ou toute revendication dépendante de celle-ci, les première et seconde faces opposées présentant chacune un périmètre incurvé sans coins.

13. Cartouche selon la revendication 1 ou toute revendication dépendante de celle-ci, ou dispositif de fourniture de vapeur selon la revendication 2 ou toute revendication dépendante de celle-ci, la première face opposée étant située dans un premier plan et la seconde face opposée étant située dans un second plan, et les premier et second plans étant légèrement inclinés l'un vers l'autre par rapport à la direction longitudinale.

14. Cartouche ou dispositif de fourniture de vapeur selon la revendication 13, le trou de sortie étant situé là où les première et seconde faces opposées sont le plus près l'une de l'autre.

15. Cartouche selon la revendication 1 ou toute revendication dépendante de celle-ci, ou dispositif de fourniture de vapeur selon la revendication 2 ou toute revendication dépendante de celle-ci, les première et seconde faces opposées étant inclinées l'une vers l'autre par rapport à la direction longitudinale d'un angle d'inclinaison compris dans la plage de 5 à 25 degrés, ou dans la plage de 10 à 20 degrés, et de préférence d'environ 15 degrés.
